# EUROPEAN PATENT APPLICATION

(11) **EP 2 386 290 A2**
(43) Date of publication of application: **16.11.2011**
(21) Application number: 11250311.5
(22) Date of filing: 15.03.2011
(51) Int. Cl.: A61K 9/00, A61K 9/06, A61K 47/32

(54) **Modulating drug release rate by controlling the kinetics of the pH transition in hydrogels**

(30) Priority: 16.03.2010 US 724458
(71) Applicant: Confluent Surgical Inc., Waltham, Massachusetts 02451 (US)
(72) Inventor: Blaskovich, Philip, Salem, MA 01970 (US); Ohri, Rachit, Framingham, MA 01701 (US); Costa, Daniel S., Somerville, MA 02143 (US)
(74) Representative: Soames, Candida Jane

(57) **Abstract**

Methods and compositions relate to modulating the release profile of drug molecules from a hydrogel by controlling the kinetics of the pH transition of the hydrogel. The hydrogel is formed by in situ polymerization and includes a drug molecule having a pKa between the pH of the formed hydrogel and the physiologic environment in which the hydrogel is placed.

## Description

### TECHNICAL FIELD

The present disclosure is related to biocompatible crosslinked hydrogels, in embodiments, to hydrogels which modulate the release profile of active pharmaceutical ingredients contained therein by controlling the kinetics of the pH transition of the hydrogel.

### BACKGROUND

Hydrogels may be used in the body for surgical applications such as sealing, adhesion prevention, or drug delivery. For drug delivery, prolonged or expedited release may be difficult to achieve in a controlled manner.

Drugs may be released by diffusion through the hydrogel to the surrounding tissue, degradation of the hydrogel itself, or a combination of both diffusion and degradation. Drug release may be influenced by formulation variables such as the physicochemical properties of the drug, including drug solubility and the method of drug incorporation, e.g., the use of encapsulation vehicles like microspheres or microcapsules. It would be advantageous to modulate drug release profiles without any manipulation of the drug molecule or compound, and without the use of excipients.

### SUMMARY

The present disclosure relates to hydrogel which modulate the release profile of active pharmaceutical ingredients contained therein, by controlling the kinetics of the pH transition of the hydrogel. The hydrogel includes a first precursor combined with a first buffer, a second precursor combined with a second buffer, and a bioactive agent. The pKa of the bioactive agent may be greater or lesser than the pH of the hydrogel, and the pH of the first buffer, the second buffer, or both, may be selected to increase or decrease the release of the bioactive agent from the hydrogel.
[0005.1] In embodiments in which the pKa of the bioactive agent is greater than the pH of the hydrogel, the pH of the first buffer, the second buffer, or both may be greater than the pKa of the bioactive agent, thereby decreasing the release of the bioactive agent from the hydrogel. Alternatively, the pH of the first and/or second buffer may be less than the pKa of the bioactive agent to increase the release of the bioactive agent from the hydrogel. In embodiments in which the pKa of the bioactive agent is less than the pH of the hydrogel, the pH of the first buffer, the second buffer, or both may be less than the pKa of the bioactive agent, thereby decreasing the release of the bioactive agent from the hydrogel. Alternatively, the pH of the first and/or second buffer may be greater than the pKa of the bioactive agent to increase the release of the bioactive agent from the hydrogel.
[0005.2] Methods of forming a hydrogel are also disclosed. A method of the present disclosure includes contacting a first hydrogel precursor with a first buffer, contacting a second hydrogel precursor with a second buffer, adding a bioactive agent to the first hydrogel precursor, the second hydrogel precursor, or both, and contacting tissue with the first and second hydrogel precursors. The first and second hydrogel precursors form a hydrogel upon contact and include a bioactive agent having a pKa between the initial pH of the formed hydrogel and the pH of the physiologic environment in which the hydrogel is placed.
[0005.3] A kit may be utilized to produce a hydrogel. The kit may include a first precursor, a second precursor, a first buffer for the first precursor, a second buffer for the second precursor, a bioactive agent, and an applicator for substantially simultaneously delivering the bioactive agent and the first and second precursors. --

### BRIEF DESCRIPTION OF THE FIGURES

The Figure is a graph depicting the effect of different molar concentrations of dibasic sodium phosphate on the in vitro release of bupivacaine from 0.75% (w/v) loaded gels.

### DETAILED DESCRIPTION

Hydrogels are described herein that are suitable for a variety of uses such as, for example, adhesives, hemostats, sealants, protective barriers, and the like. As such, the hydrogel may function as a tissue adhesive, tissue sealant, drug delivery vehicle, wound covering agent, barrier to prevent postoperative adhesions, or a covering of inflamed or injured sites. The hydrogel may also be applied as a bolus that fills a void or lumen and/or as a coating that conforms to a tissue surface or to a three dimensional shape.

These hydrogels may have good adhesion to tissues, can be formed in-situ, are optionally biodegradable, and can exhibit mechanical properties adequate to withstand strains which may be caused by movement of the patient, shifting of tissues, hydrostatic forces present in the tissue, and the like. At the same time, a high water content in the hydrogel can be useful for biocompatibility.

Certain hydrogel properties can be useful, such as adhesion to a variety of tissues, fast setting times to enable a surgeon to accurately and conveniently place the hydrogels, high water content for biocompatibility, mechanical strength for use as sealants, and/or toughness to resist destruction after placement. Synthetic materials that are readily sterilized and avoid the dangers of disease transmission involved in the use of natural materials can also be used.

### Hydrogel Systems Overview

The components for forming hydrogels on or in tissues include, for example, in situ forming materials. The in situ forming material may include a single precursor or multiple precursors that form "in situ", meaning formation occurs at a tissue in a living animal or human body. In general, this may be accomplished by having a precursor that can be activated at the time of application to a tissue to form an in situ forming material, in embodiments a hydrogel.

In situ forming materials may be formed either through covalent, ionic or hydrophobic bonds. Physical (non-covalent) crosslinks may result from complexation, hydrogen bonding, desolvation, Van der Waals interactions, ionic bonding, combinations thereof, and the like, and may be initiated by mixing two precursors that are physically separated until combined in situ, or as a consequence of a prevalent condition in the physiological environment, including temperature, pH, ionic strength, combinations thereof, and the like. Chemical (covalent) crosslinking may be accomplished by any of a number of mechanisms, including free radical polymerization, condensation polymerization, anionic or cationic polymerization, step growth polymerization, electrophile-nucleophile reactions, combinations thereof, and the like.

In some embodiments, in situ forming material systems may include biocompatible multi-precursor systems that spontaneously crosslink when the precursors are mixed, but wherein the two or more precursors are individually stable for the duration of the deposition process. Such systems include, for example for a hydrogel, a first precursor including macromers that are di or multifunctional amines and a second precursor including di or multifunctional oxirane containing moieties.

Some embodiments of forming an in situ forming material involve mixing precursors that crosslink quickly after application to a surface, e.g., on a tissue of a patient, to form an in situ forming material.

The crosslinking reaction leading to gelation can occur, in some embodiments, within a time from about 1 second to about 5 minutes, in embodiments from about 3 seconds to about 1 minute; persons of ordinary skill in these arts will immediately appreciate that all ranges and values within these explicitly stated ranges are contemplated. In some cases gelation may occur in less than 10 seconds.

The precursors may be placed into solution prior to use, with the solution being delivered to the patient. Solutions suitable for use in accordance with the present disclosure include those that may be used to form implants in lumens or voids. Where two solutions are employed, each solution may contain one precursor of an in situ forming material which forms upon on contact. The solutions may be separately stored and mixed when delivered to tissue.

Additionally, any solutions utilized as part of the in situ forming material system should not contain harmful or toxic solvents. In embodiments, the precursor(s) may be substantially soluble in a solvent such as water to allow application in a physiologically-compatible solution, such as buffered isotonic saline. Water-soluble coatings may form thin films, but in embodiments may also form three-dimensional gels of controlled thickness. The gel may also be biodegradable, so that it does not have to be retrieved from the body. Biodegradability, as used herein, refers to the predictable disintegration of the coating into molecules small enough to be metabolized or excreted under normal physiological conditions.

Properties of the in situ forming material system may be selected according to the intended application. For example, if the in situ forming material is to be used to temporarily occlude a reproductive organ, such as a fallopian tube, it may be desirable that the in situ forming material system undergo significant swelling and be biodegradable. Alternatively, the in situ forming material may have thrombotic properties, or its precursors may react with blood or other body fluids to form a coagulum.

Other applications may require different characteristics of the in situ forming material system. Generally, the materials should be selected on the basis of exhibited biocompatibility and lack of toxicity.

Certain properties of the in situ forming material can be useful, including adhesion to a variety of tissues, desirable setting times to enable a surgeon to accurately and conveniently place the in situ forming materials, high water content for biocompatibility, which may be relevant for hydrogels, mechanical strength for use in sealants, and/or toughness to resist destruction after placement. Synthetic materials that are readily sterilized and avoid the dangers of disease transmission involved in the use of natural materials may thus be used. Indeed, certain in situ polymerizable hydrogels made using synthetic precursors are within the purview of those skilled in the art, e.g., as used in commercially available products such as FOCALSEAL^{®} (Genzyme, Inc.), COSEAL^{®} (Angiotech Pharmaceuticals), and DURASEAL^{®} (Confluent Surgical, Inc). Other known hydrogels include, for example, those disclosed in U.S. Patent Nos. 6,656,200; 5,874,500; 5,543,441; 5,514,379; 5,410,016; 5,162,430; 5,324,775; 5,752,974; and 5,550,187.

As noted above, in situ forming materials may be made from one or more precursors. The precursor may be, e.g., a monomer or a macromer. One type of precursor may have a functional group that is ethylenically unsaturated. An ethylenically unsaturated functional group may be polymerized using an initiator to start the reaction. Precursors with at least two ethylenically unsaturated functional groups may form crosslinked polymers. Some compositions have certain precursors with only one such functional group and additional crosslinker precursors with a plurality of functional groups for crosslinking the precursors. Ethylenically unsaturated functional groups may be polymerized by various techniques, e.g., free radical, condensation, or addition polymerization.

In situ forming materials may thus be formed from one precursor (as by free radical polymerization), two precursors, or made with three or more precursors, with one or more of the precursors participating in crosslinking to form the in situ forming material.

Other precursors which may be used to form a hydrogel may have a functional group that is an electrophile or nucleophile. Electrophiles react with nucleophiles to form covalent bonds. Covalent crosslinks or bonds refer to chemical groups formed by reaction of functional groups on different polymers that serve to covalently bind the different polymers to each other. In certain embodiments, a first set of electrophilic functional groups on a first precursor may react with a second set of nucleophilic functional groups on a second precursor. When the precursors are mixed in an environment that permits reaction (e.g., as relating to pH or solvent), the functional groups react with each other to form covalent bonds. The precursors become crosslinked when at least some of the precursors can react with more than one other precursor. For instance, a precursor with two functional groups of a first type may be reacted with a crosslinking precursor that has at least three functional groups of a second type capable of reacting with the first type of functional groups.

As noted above, an in situ forming material may be a hydrogel. In embodiments the hydrogel may be formed from single precursors or multiple precursors. For example, where the hydrogel is formed from multiple precursors, for example two precursors, the precursors may be referred to as a first and second hydrogel precursor. The terms "first hydrogel precursor" and "second hydrogel precursor" each mean a polymer, functional polymer, macromolecule, small molecule, or crosslinker that can take part in a reaction to form a network of crosslinked molecules, e.g., a hydrogel.

In embodiments, each of the first and second hydrogel precursors includes only one category of functional groups, either only nucleophilic groups or only electrophilic functional groups, so long as both nucleophilic and electrophilic precursors are used in the crosslinking reaction. Thus, for example, if the first hydrogel precursor has nucleophilic functional groups such as amines, the second hydrogel precursor may have electrophilic functional groups such as N-hydroxysuccinimides. On the other hand, if first hydrogel precursor has electrophilic functional groups such as sulfosuccinimides, then the second hydrogel precursor may have nucleophilic functional groups such as amines or thiols. Thus, functional polymers such as proteins, poly(allyl amine), styrene sulfonic acid, or amine-terminated di- or multifunctional poly(ethylene glycol) ("PEG") can be used.

The first and second hydrogel precursors may have biologically inert and water soluble cores. When the core is a polymeric region that is water soluble, suitable polymers that may be used include: polyethers, for example, polyalkylene oxides such as polyethylene glycol("PEG"), polyethylene oxide ("PEO"), polyethylene oxide-co-polypropylene oxide ("PPO"), co-polyethylene oxide block or random copolymers, and polyvinyl alcohol ("PVA"); poly(vinyl pyrrolidinone) ("PVP"); poly(amino acids); poly (saccharides), such as dextran; chitosan; alginates; carboxymethylcellulose; oxidized cellulose; hydroxyethylcellulose; hydroxymethylcellulose; hyaluronic acid; and proteins such as albumin, collagen, casein, and gelatin. The polyethers, and more particularly poly(oxyalkylenes) or poly(ethylene glycol) or polyethylene glycol, may be utilized in some embodiments. When the core is small in molecular nature, any of a variety of hydrophilic functionalities can be used to make the first and second hydrogel precursors water soluble. In embodiments, functional groups like hydroxyl, amine, sulfonate and carboxylate, which are water soluble, maybe used to make the precursor water soluble. For example, the N-hydroxysuccinimide ("NHS") ester of subaric acid is insoluble in water, but by adding a sulfonate group to the succinimide ring, the NHS ester of subaric acid may be made water soluble, without affecting its reactivity towards amine groups.

In embodiments, at least one of the first and second hydrogel precursors is a cross-linker. In embodiments, at least one of the first and second hydrogel precursors is a macromolecule, and may be referred to herein as a "functional polymer".

Each of the first and second hydrogel precursors may be multifunctional, meaning that it may include two or more electrophilic or nucleophilic functional groups, such that, for example, a nucleophilic functional group on the first hydrogel precursor may react with an electrophilic functional group on the second hydrogel precursor to form a covalent bond. At least one of the first or second hydrogel precursors includes more than two functional groups, so that, as a result of electrophilic-nucleophilic reactions, the precursors combine to form cross-linked polymeric products.

In embodiments, a multifunctional nucleophilic polymer such as trilysine may be used as a first hydrogel precursor and a multifunctional electrophilic polymer such as a multi-arm PEG functionalized with multiple NHS groups may be used as a second hydrogel precursor. The multi-arm PEG functionalized with multiple NHS groups can, for example, have four, six or eight arms and a molecular weight of from about 5,000 to about 25,000. Other examples of suitable first and second hydrogel precursors are described in U.S. Patent Nos. 6,152,943; 6,165,201; 6,179,862; 6,514,534; 6,566,406; 6,605,294; 6,673,093; 6,703,047; 6,818,018; 7,009,034; and 7,347,850, the entire disclosures of each of which are incorporated herein by reference.

In embodiments, one or more precursors having biodegradable linkages present in between functional groups may be included to make the hydrogel biodegradable or absorbable. In some embodiments, these linkages may be, for example, esters, which may be hydrolytically degraded in physiological solution. The use of such linkages is in contrast to protein linkages that may be degraded by proteolytic action. A biodegradable linkage optionally also may form part of a water soluble core of one or more of the precursors. Alternatively, or in addition, functional groups of precursors may be chosen such that the product of the reaction between them results in a biodegradable linkage. For each approach, biodegradable linkages may be chosen such that the resulting biodegradable biocompatible crosslinked polymer degrades or is absorbed in a desired period of time. Generally, biodegradable linkages may be selected that degrade the hydrogel under physiological conditions into non-toxic or low toxicity products.

In embodiments an in situ forming material may also include an initiator. An initiator may be any precursor or group capable of initiating a polymerization reaction for the formation of the in situ forming material.

### Preparation of Polymers

The reaction conditions for forming crosslinked polymeric hydrogels will depend on the nature of the functional groups. In embodiments, reactions are conducted in buffered aqueous solutions at a pH of about 3 to about 12, in embodiments from about 5 to about 9. Buffers include, for example, sodium borate buffer (pH 10) and triethanol amine buffer (pH 7). In some embodiments, organic solvents such as ethanol or isopropanol may be added to improve the reaction speed or to adjust the viscosity of a given formulation.

When the crosslinker and functional polymers are synthetic (for example, when they are based on polyalkylene oxide), it may be desirable to use molar equivalent quantities of the reactants. In some cases, molar excess of a crosslinker may be added to compensate for side reactions such as reactions due to hydrolysis of the functional group.

Synthetic crosslinked gels degrade due to hydrolysis of the biodegradable region. The degradation of gels containing synthetic peptide sequences will depend on the specific enzyme and its concentration. In some cases, a specific enzyme may be added during the crosslinking reaction to accelerate the degradation process.

When choosing the crosslinker and crosslinkable polymer, at least one of the polymers may have more than two functional groups per molecule and at least one degradable region, if it is desired that the resultant biocompatible crosslinked polymer be biodegradable. In embodiments, each biocompatible crosslinked polymer precursor may have more than two functional groups, and in some embodiments, more than four functional groups.

The crosslinking density of the resultant biocompatible crosslinked polymer may be controlled by the overall molecular weight of the crosslinker and functional polymer and the number of functional groups available per molecule. A lower molecular weight between crosslinks, such as 600 Da, will give much higher crosslinking density as compared to a higher molecular weight, such as 10,000 Da. Elastic gels may be obtained with higher molecular weight functional polymers with molecular weights of more than 3,000 Da.

The crosslinking density may also be controlled by the overall percent solids of the crosslinker and functional polymer solutions. Increasing the percent solids increases the probability that an electrophilic group will combine with a nucleophilic group prior to inactivation by hydrolysis. Yet another method to control crosslink density is by adjusting the stoichiometry of nucleophilic groups to electrophilic groups. A one to one ratio may lead to the highest crosslink density, however, other ratios of reactive functional groups (e.g., electrophile:nucleophile) are envisioned to suit a desired formulation.

Biodegradable crosslinkers or small molecules as described above may be reacted with proteins, such as albumin, other serum proteins, or serum concentrates to generate crosslinked polymeric networks. Generally, aqueous solutions of crosslinkers may be mixed with concentrated solutions of proteins to produce a crosslinked hydrogel. The reaction may be accelerated by adding a buffering agent, e.g., borate buffer or triethanol amine, during the crosslinking step.

The resulting crosslinked hydrogel's degradation depends on the degradable segment in the crosslinker as well as degradation by enzymes. In the absence of any degradable enzymes, the crosslinked polymer may degrade solely by hydrolysis of the biodegradable segment. In embodiments in which polyglycolate is used as the biodegradable segment, the crosslinked polymer may degrade in from about 1 day to about 30 days depending on the crosslinking density of the network. Similarly, in embodiments in which a polycaprolactone based crosslinked network is used, degradation may occur over a period of from about 1 month to about 8 months. The degradation time generally varies according to the type of degradable segment used, in the following order: polyglycolate<polylactate<polytrimethylene carbonate<polycaprolactone. Thus, it is possible to construct a hydrogel with a desired degradation profile, from a few days to months, using a proper degradable segment.

The hydrophobicity generated by biodegradable blocks such as oligohydroxy acid blocks or the hydrophobicity of PPO blocks in PLURONIC or TETRONIC polymers may be helpful in dissolving small organic drug molecules. Other properties which will be affected by incorporation of biodegradable or hydrophobic blocks include: water absorption, mechanical properties and thermosensitivity.

### In situ Polymerization

Formulations may be prepared that are suited to make precursor crosslinking reactions occur in situ. In general, this may be accomplished by having a precursor that can be activated at the time of application to a tissue to form a crosslinked hydrogel. Activation can be made before, during, or after application of the precursor to the tissue, provided that the precursor is allowed to conform to the tissue's shape before crosslinking and associated gelation is otherwise too far advanced. Activation includes, for instance, triggering a polymerization process, initiating a free radical polymerization, or mixing precursors with functional groups that react with each other. Thus, in situ polymerization includes activation of chemical moieties to form covalent bonds to create an insoluble material, e.g., a hydrogel, at a location where the material is to be placed on, within, or both on and within, a patient. In situ polymerizable polymers may be prepared from precursors that can be reacted such that they form a polymer within the patient. Thus precursors with electrophilic functional groups can be mixed or otherwise activated in the presence of precursors with nucleophilic functional groups. In other embodiments, precursors with ethylenically unsaturated groups can be initiated to polymerize in situ on the tissue of a patient.

With respect to coating a tissue, and without limiting the present disclosure to a particular theory of operation, it is believed that reactive precursor species that crosslink quickly after contacting a tissue surface may form a three dimensional structure that is mechanically interlocked with the coated tissue. This interlocking contributes to adherence, intimate contact, and continuous coverage of the coated region of the tissue. The crosslinking reaction leading to gelation can occur, in some embodiments within a time from about 1 second to about 5 minutes, in embodiments from about 3 seconds to about 1 minute; persons of ordinary skill in these arts will immediately appreciate that all ranges and values within these explicitly stated ranges are contemplated. For example, in embodiments, the in situ gelation time of hydrogels according to the present disclosure is less than about 20 seconds, and in some embodiments, less than about 10 seconds, and in yet other embodiments less than about 5 seconds. In embodiments where electrophilic precursors are used, such precursors may react with free amines in tissue, thereby serving as a means for attaching the hydrogel to tissue.

### Visualization Agents

The precursor and/or the crosslinked polymer may contain visualization agents to improve their visibility during surgical procedures. Visualization agents may be selected from a variety of non-toxic colored substances, such as dyes, suitable for use in implantable medical devices. Suitable dyes are within the purview of those skilled in the art and may include, for example, a dye for visualizing a thickness of the hydrogel as it is formed in situ, e.g., as described in U.S. Patent No. 7,009,034. In some embodiments, a suitable dye may include, for example, FD&C Blue #1, FD&C Blue #2, FD&C Blue #3, FD&C Blue #6, D&C Green #6, methylene blue, indocyanine green, other colored dyes, and combinations thereof. It is envisioned that additional visualization agents may be used such as fluorescent compounds (e.g., flurescein or eosin), x-ray contrast agents (e.g., iodinated compounds), ultrasonic contrast agents, and MRI contrast agents (e.g., Gadolinium containing compounds).

The visualization agent may be present in either a crosslinker or functional polymer solution. The colored substance may or may not become incorporated into the biocompatible crosslinked polymer. In embodiments, however, the visualization agent does not have a functional group capable of reacting with the crosslinker or functional polymer.

The visualization agent may be used in small quantities, in embodiments less than 1% weight/volume, and in other embodiments less that 0.01 % weight/volume and in yet other embodiments less than 0.001% weight/volume concentration.

### Delivery of Bioactive Agents

The subject precursors, such as the crosslinkers and functional polymers described above, as well as their reaction products, may be used for drug therapy or delivery of bioactive agents. As used herein, a bioactive agent includes any active pharmaceutical ingredient or drug that provides a therapeutic or prophylactic effect, a compound that affects or participates in tissue growth or cell differentiation, a compound that may be able to invoke a biological action such as an immune response or that may play any other role in one or more biological processes may be used. Biologically active agents or drug compounds that may be added and delivered from the crosslinked polymer or gel include: proteins, glycosaminoglycans, carbohydrates, nucleic acid, and inorganic and organic biologically active compounds.

Examples of drugs and alternative forms of these drugs such as salt forms, free acid forms, free base forms, and hydrates include: antimicrobials (e.g., cephalosporins such as cefazolin sodium, cephradine, cefaclor, cephapirin sodium, ceftizoxime sodium, cefoperazone sodium, cefotetan disodium, cefuroxime azotil, cefotaxime sodium, cefadroxil monohydrate, cephalexin, cephalothin sodium, cephalexin hydrochloride monohydrate, cefamandole nafate, cefoxitin sodium, cefonicid sodium, ceforanide, ceftriaxone sodium, ceftazidime, cefadroxil, cephradine, and cefuroxime sodium); penicillins (e.g., ampicillin, amoxicillin, penicillin G benzathine, cyclacillin, ampicillin sodium, penicillin G potassium, penicillin V potassium, piperacillin sodium, oxacillin sodium, bacampicillin hydrochloride, cloxacillin sodium, ticarcillin disodium, azlocillin sodium, carbenicillin indanyl sodium, penicillin G procaine, methicillin sodium, and nafcillin sodium); erythromycins (e.g., erythromycin ethylsuccinate, erythromycin, erythromycin estolate, erythromycin lactobionate, erythromycin stearate, and erythromycin ethylsuccinate); and tetracyclines (e.g., tetracycline hydrochloride, doxycycline hyclate, and minocycline hydrochloride, azithromycin, and clarithromycin); analgesics/antipyretics (e.g., aspirin, acetaminophen, ibuprofen, naproxen sodium, buprenorphine, propoxyphene hydrochloride, propoxyphene napsylate, meperidine hydrochloride, hydromorphone hydrochloride, morphine, oxycodone, codeine, dihydrocodeine bitartrate, pentazocine, hydrocodone bitartrate, levorphanol, diflunisal, trolamine salicylate, nalbuphine hydrochloride, mefenamic acid, butorphanol, choline salicylate, butalbital, phenyltoloxamine citrate, diphenhydramine citrate, methotrimeprazine, cinnamedrine hydrochloride, and meprobamate); anesthetics; antiepileptics; antihistamines; non-steroidal anti-inflammatories (e.g., indomethacin, ketoprofen, flurbiprofen, naproxen, ibuprofen, ramifenazone, and piroxicam); steroidal anti-inflammatories (e.g., cortisone, dexamethasone, fluazacort, celecoxib, rofecoxib, hydrocortisone, prednisolone, and prednisone); cardiovascular drugs (e.g., coronary vasodilators and nitroglycerin); diagnostic agents; cholinomimetics; antimuscarinics; muscle relaxants; adrenergic neuron blockers; neurotransmitters; antineoplastics (e.g., cyclophosphamide, actinomycin, bleomycin, daunorubicin, doxorubicin hydrochloride, epirubicin, mitomycin, methotrexate, fluorouracil, carboplatin, carmustine (BCNU), methyl-CCNU, cisplatin, etoposide, camptothecin and derivatives thereof, phenesterine, paclitaxel and derivatives thereof, docetaxel and derivatives thereof, vinblastine, vincristine, tamoxifen, and piposulfan,); immunogenic agents; immunosuppressants (e.g., cyclosporine, azathioprine, mizoribine, and FK506 (tacrolimus)); gastrointestinal drugs; diuretics; lipids; lipopolysaccharides; polysaccharides; enzymes; non-steroidal antifertility agents; parasympathomimetic agents; psychotherapeutic agents; psychoactive drugs; tranquilizers; decongestants; sedative hypnotics (e.g., barbiturates such as pentobarbital and secobarbital); and benzodiazapines such as flurazepam hydrochloride, triazolam, and midazolam); steroids; sulfonamides; vitamins; antimalarials; anti-migraine agents (e.g., ergotamine, propanolol, isometheptene mucate, and dichloralphenazone); anti-parkinson agents (e.g., L-Dopa and ethosuximide); antitussives; bronchodilators (e.g., sympathomimetics such as epinephrine hydrochloride, metaproterenol sulfate, terbutaline sulfate, isoetharine, isoetharine mesylate, isoetharine hydrochloride, albuterol sulfate, albuterol, bitolterolmesylate, isoproterenol hydrochloride, terbutaline sulfate, epinephrine bitartrate, metaproterenol sulfate, epinephrine, and epinephrine bitartrate); anticholinergic agents (e.g., oxybutynin and ipratropium bromide); xanthines (e.g., aminophylline, dyphylline, metaproterenol sulfate, and aminophylline); mast cell stabilizers (e.g., cromolyn sodium); inhalant corticosteroids (e.g., beclomethasone dipropionate (BDP), and beclomethasone dipropionate monohydrate; salbutamol; ipratropium bromide; budesonide; ketotifen; salmeterol; xinafoate; terbutaline sulfate; triamcinolone; theophylline; nedocromil sodium; metaproterenol sulfate; flunisolide; and fluticasone proprionate); angiogenic agents; anti-angiogenic agents; alkaloids; analgesics; narcotics (e.g., codeine, dihydrocodeinone, meperidine, morphine, and the like); opoid receptor antagonists (e.g., naltrexone and naloxone); anti-cancer agents; chemotherapeutic drugs; anti-convulsants; anti-emetics (e.g., meclizine hydrochloride, nabilone, prochlorperazine, dimenhydrinate, promethazine hydrochloride, thiethylperazine, and scopolamine); antihistimines (e.g., hydroxyzine, diphenhydramine, chlorpheniramine, brompheniramine maleate, cyproheptadine hydrochloride, terfenadine, clemastine fumarate, triprolidine, carbinoxamine, diphenylpyraline, phenindamine, azatadine, tripelennamine, dexchlorpheniramine maleate, and methdilazine); antiinflammatory agents (e.g., hormonal agents, hydrocortisone, non-hormonal agents, allopurinol, indomethacin, phenylbutzone and the like); prostaglandins and cytotoxic drugs; drugs affecting reproductive organs; estrogens; antibacterials (e.g., amikacin sulfate, aztreonam, chloramphenicol, chloramphenicol palirtate, ciprofloxacin, clindamycin, clindamycin palmitate, clindamycin phosphate, metronidazole, metronidazole hydrochloride, gentamicin sulfate, lincomycin hydrochloride, tobramycin sulfate, vancomycin hydrochloride, polymyxin B sulfate, colistimethate sodium, and colistin sulfate); antibodies; antibiotics (e.g., neomycin, streptomycin, chloramphenicol, cephalosporin, ampicillin, penicillin, tetracycline, and ciprofloxacin); anti-fungals (e.g., griseofulvin, ketoconazole, itraconizole, amphotericin B, nystatin, and candicidin); anti-virals (e.g., interferon alpha, beta or gamma, zidovudine, amantadine hydrochloride, ribavirin, and acyclovir); anticoagulants (e.g., heparin, heparin sodium, and warfarin sodium); antidepressants (e.g., nefopam, oxypertine, doxepin, amoxapine, trazodone, amitriptyline, maprotiline, phenylzine, desipramine, nortriptyline, tranylcypromine, fluoxetine, doxepin, imipramine, imipramine pamoate, isocarboxazid, trimipramine, and protriptyline); immunological agents; antiasthamatics (e.g., ketotifen and traxanox); antidiabetics (e.g., biguanides and sulfonylurea derivatives); antihypertensive agents (e.g., propanolol, propafenone, oxyprenolol, nifedipine, reserpine, trimethaphan, phenoxybenzamine, pargyline hydrochloride, deserpidine, diazoxide, guanethidine monosulfate, minoxidil, rescinnamine, sodium nitroprusside, rauwolfia serpentina, alseroxylon, and phentolamine); antianxiety agents (e.g., lorazepam, buspirone, prazepam, chlordiazepoxide, oxazepam, clorazepate dipotassium, diazepam, hydroxyzine pamoate, hydroxyzine hydrochloride, alprazolam, droperidol, halazepam, chlormezanone, and dantrolene); antianginal agents (e.g., beta-adrenergic blockers; calcium channel blockers (e.g., nifedipine and diltiazem); nitrates (e.g., nitroglycerin, isosorbide dinitrate, pentaerythritol tetranitrate, and erythrityl tetranitrate); antipsychotic agents (e.g., haloperidol, loxapine succinate, loxapine hydrochloride, thioridazine, thioridazine hydrochloride, thiothixene, fluphenazine, fluphenazine decanoate, fluphenazine enanthate, trifluoperazine, chlorpromazine, perphenazine, lithium citrate, and prochlorperazine); antimanic agents (e.g., lithium carbonate); antiarrhythmics (e.g., bretylium tosylate, esmolol, verapamil, amiodarone, encainide, digoxin, digitoxin, mexiletine, disopyramide phosphate, procainamide, quinidine sulfate, quinidine gluconate, quinidine polygalacturonate, flecainide acetate, tocainide, and lidocaine); antiarthritic agents (e.g., phenylbutazone, sulindac, penicillanine, salsalate, piroxicam, azathioprine, indomethacin, meclofenamate, gold sodium thiomalate, ketoprofen, auranofin, aurothioglucose, and tolmetin sodium); antigout agents (e.g., colchicine and allopurinol); thrombolytic agents (e.g., urokinase, streptokinase, and alteplase); antifibrinolytic agents (e.g., aminocaproic acid); hemorheologic agents (e.g., pentoxifylline); antiplatelet agents (e.g., aspirin); anticonvulsants (e.g., valproic acid, divalproex sodium, phenyloin, phenyloin sodium, clonazepam, primidone, phenobarbitol, carbamazepine, amobarbital sodium, methsuximide, metharbital, mephobarbital, mephenyloin, phensuximide, paramethadione, ethotoin, phenacemide, secobarbitol sodium, clorazepate dipotassium, and trimethadione); agents useful for calcium regulation (e.g., calcitonin and parathyroid hormone); anti-infectives (e.g., GM-CSF); steroidal compounds and hormones (e.g., androgens such as danazol, testosterone cypionate, fluoxymesterone, ethyltestosterone, testosterone enathate, methyltestosterone, fluoxymesterone, and testosterone cypionate; estrogens such as estradiol, estropipate, and conjugated estrogens); progestins (e.g., methoxyprogesterone acetate and norethindrone acetate); corticosteroids (e.g., triamcinolone, betamethasone, betamethasone sodium phosphate, dexamethasone, dexamethasone sodium phosphate, dexamethasone acetate, prednisone, methylprednisolone acetate suspension, triamcinolone acetonide, methylprednisolone, prednisolone sodium phosphate, methylprednisolone sodium succinate, hydrocortisone sodium succinate, triamcinolone hexacetonide, hydrocortisone, hydrocortisone cypionate, prednisolone, fludrocortisone acetate, paramethasone acetate, prednisolone tebutate, prednisolone acetate, prednisolone sodium phosphate, and hydrocortisone sodium succinate); and thyroid hormones (e.g., levothyroxine sodium); hypoglycemic agents (e.g., human insulin, purified beef insulin, purified pork insulin, glyburide, chlorpropamide, glipizide, tolbutamide, and tolazamide); hypolipidemic agents (e.g., clofibrate, dextrothyroxine sodium, probucol, pravastitin, atorvastatin, lovastatin, and niacin); agents useful for erythropoiesis stimulation (e.g., erythropoietin); and antiulcer/antireflux agents (e.g., famotidine, cimetidine, and ranitidine hydrochloride).

Other examples of suitable biologically active agents include viruses and cells; peptides; polypeptides and proteins; analogs; bacteriophages; muteins and active fragments thereof, such as immunoglobulins, antibodies, and cytokines (e.g., lymphokines, monokines, and chemokines); blood clotting factors; hemopoietic factors; interleukins (e.g., IL-2, IL-3, IL-4, IL-6); interferons (e.g., β-IFN, (α-IFN and γ-IFN)); erythropoietin; nucleases; tumor necrosis factor; colony stimulating factors (e.g., GCSF, GM-CSF, and MCSF); insulin; anti-tumor agents and tumor suppressors; blood proteins; gonadotropins (e.g., FSH, LH, CG, etc.); hormones and hormone analogs (e.g., growth hormone); vaccines (e.g., tumoral, bacterial and viral antigens); somatostatin; antigens; blood coagulation factors; growth factors (e.g., nerve growth factor and insulin-like growth factor); proteins (e.g., DNase, alginase, superoxide dismutase, and lipase); protein inhibitors, protein antagonists, and protein agonists; nucleic acids (e.g., sense or antisense nucleic acids encoding any therapeutically useful protein, including any of the proteins described herein, DNA, and RNA); oligonucleotides; polynucleotides; and ribozymes.

Other bioactive agents useful in the compositions and methods described herein include mitotane, halonitrosoureas, anthrocyclines, ellipticine, ceftazidime, oxaprozin, valacyclovir, famciclovir, flutamide, enalapril, mefformin, itraconazole, gabapentin, fosinopril, tramadol, acarbose, lorazepan, follitropin, omeprazole, lisinopril, tramsdol, levofloxacin, zafirlukast, granulocyte stimulating factor, nizatidine, bupropion, perindopril, erbumine, adenosine, alendronate, alprostadil, betaxolol, bleomycin sulfate, dexfenfluramine, fentanyl, gemcitabine, glatiramer acetate, granisetron, lamivudine, mangafodipir trisodium, mesalamine, metoprolol fumarate, miglitol, moexipril, monteleukast, octreotide acetate, olopatadine, paricalcitol, somatropin, sumatriptan succinate, tacrine, nabumetone, trovafloxacin, dolasetron, finasteride, isradipine, tolcapone, enoxaparin, fluconazole, lansoprazole, pamidronate, didanosine, diclofenac, cisapride, venlafaxine, troglitazone, fluvastatin, losartan, imiglucerase, donepezil, olanzapine, valsartan, fexofenadine, adapalene, doxazosin mesylate, mometasone furoate, ursodiol, felodipine, nefazodone hydrochloride, valrubicin, albendazole, medroxyprogesterone acetate, nicardipine hydrochloride, zolpidem tartrate, rubitecan, amlodipine besylate/benazepril hydrochloride, paroxetine hydrochloride, podofilox, pramipexole dihydrochloride, quetiapine fumarate, candesartan, cilexetil, ritonavir, busulfan, flumazenil, risperidone, carbemazepine, carbidopa, levodopa, ganciclovir, saquinavir, amprenavir, sertraline hydrochloride, clobustasol, diflucortolone, halobetasolproprionate, sildenafil citrate, chlorthalidone, imiquimod, simvastatin, citalopram, irinotecan hydrochloride, sparfloxacin, efavirenz, tamsulosin hydrochloride, mofafinil, letrozole, terbinafine hydrochloride, rosiglitazone maleate, lomefloxacin hydrochloride, tirofiban hydrochloride, telmisartan, diazapam, loratadine, toremifene citrate, thalidomide, dinoprostone, mefloquine hydrochloride, trandolapril, mitoxantrone hydrochloride, tretinoin, etodolac, nelfinavir mesylate, indinavir, nifedipine, cefuroxime, and nimodipine.

### Applicators

The precursors may be placed into solution prior to use, with the solution being delivered to the patient. The hydrogel system solutions should not contain harmful or toxic solvents. In embodiments, the precursors may be substantially soluble in water to allow application in a physiologically-compatible solution, such as buffered isotonic saline. One may use a dual syringe or similar device to apply the precursor solutions, such as those described in U.S. Patent Nos. 4,874,368; 4,631,055; 4,735,616; 4,359,049; 4,978,336; 5,116,315; 4,902,281; 4,932,942; 6,179,862; 6,673,093; 6,152,943; and 7,347,850.

Generally, two or more crosslinkable components may be applied via a sprayer to the tissue to form a coating in situ. For example, two crosslinkable precursor solutions, each containing one component of a co-initiating system capable of crosslinking when mixed together, may be placed in separate chambers of the sprayer. When the sprayer is activated, the emergent spray contacts tissue, resulting in mixing and crosslinking of the two solutions to form a coating (for example a hydrogel) on the tissue surface.

In embodiments, the sprayer includes separate spray nozzles for each of two or more crosslinkable solutions, with each nozzle surrounded by a separate or common gas flow outlet. The crosslinkable solutions are stored in separate compartments, e.g., a multi-cylinder syringe, and communicated under pressure to the spray nozzles. In the presence of gas flow through the gas flow outlets, the crosslinkable solutions are atomized and mixed in the gas flow to form a spray, which may be used to coat tissue. In certain embodiments, a CO₂ gas cartridge is reversibly or permanently mounted on the device to facilitate delivery of the precursors.

Certain embodiments include combining a suction-irrigation apparatus with a precursor delivery device. An advantage of such a combination is that the tissue can be cleansed of clotted blood and adhesioniogenic materials and allows placement of a hydrogel barrier with a single device.

### Controlling the drug-release profile of Hydrogels

Changing the kinetics of the pH transition within the hydrogel microenvironment provides an opportunity for the modulation of the release profile of several drug molecules. This modulation may be achieved by the addition of appropriate buffers, or additional chemical agents into the buffers, such as alkalizers and acidifers, which can change the kinetics of the pH transition from alkaline or acidic to physiologic. As used herein, an acidifier is any component or material which may lower the pH of a composition, and an alkalizer is any component or material which may raise the pH of a composition.

There are several variables which can impact the rate of release of a therapeutic molecule from a hydrogel. For example, the inherent water solubility or hydrophobicity of the therapeutic molecule can impact its rate of diffusion (and therefore rate of release) outside the hydrogel. In addition, the pH of the surrounding physiological environment can impact the rate of pH transition within the gel. The rate of pH transition can be further affected by the inclusion of buffers of varying compositions and strengths which participate in gel formation, as well as the presence of alkalizers or acidizers which can slow down or speed up pH transition kinetics.

In addition to the rate of release, the overall release of a therapeutic molecule from a hydrogel, i.e., the total amount of therapeutic molecule released from the hydrogel, may be similarly increased or decreased utilizing the methods of the present disclosure. In embodiments, the amount of therapeutic molecule released from the hydrogel may be measured at a point in time after administration, with compositions of the present disclosure having an increased or decreased amount of release at that point in time. The increase or decrease may be dependent upon the hydrogel utilized, the therapeutic molecule utilized, combinations thereof, and the like, and may be tailored depending upon the desired treatment.

For example, for the treatment of pain, compositions of the present disclosure may have an increased or decreased amount of release of therapeutic molecule at a time of from about 30 minutes to about 4 days after administration, in embodiments from about 1 day to about 2.5 days after administration. Where the therapeutic molecule is utilized to prevent and/or treat infection, for example an antibiotic, the compositions of the present disclosure may have an increased or decreased amount of release of therapeutic molecule at a time of from about 1 day to about 10 days after administration, in embodiments from about 2 days to about 7 days after administration. Other therapeutic molecules, including chemotherapeutics, may have an increased or decreased amount of release of therapeutic molecule at a time of from about 1 day to about 60 days after administration, in embodiments from about 3 days to about 45 days after administration. Yet other therapeutic molecules may have an increased or decreased amount of release of therapeutic molecule at a time of from about 30 days to about 120 days after administration, in embodiments from about 45 days to about 90 days after administration.

Multiple hydrogels, and/or hydrogels having multiple phases, may also be utilized to administer more than one therapeutic molecule. Thus, for example, a multiphase composition of the present disclosure may have an increased or decreased amount of release of an analgesic at a time of from about 30 minutes to about 4 days after administration, in embodiments from about 1 day to about 2.5 days after administration, and an increased or decreased amount of release of an antibiotic at a time of from about 1 day to about 10 days after administration, in embodiments from about 2 days to about 7 days after administration.

Buffers may be used to maintain a given pH range and to resist rapid pH changes. Buffers may also have a pH suitable for controlling and/or adjusting the pH of the hydrogel without the addition of any additional components. In embodiments, the buffers may include alkalizers and/or acidifiers for controlling and/or adjusting the pH of the hydrogel. In embodiments, the alkalizers and/or acidifiers may be utilized alone without the addition of any additional components to function as buffers.

Alkalizers are capable of consuming protons from the hydrogel or releasing hydroxyl groups into the hydrogel in an amount effective to raise the pH of the hydrogel while it is placed in the physiologic environment. Alkalizers include, for example, magnesium oxide, sodium hydroxide, potassium hydroxide, sodium carbonate, sodium bicarbonate, bentonite, dibasic sodium phosphate, dipotassium phosphate, arginine. Other alkalizers, sometimes referred to herein, in embodiments, as alkalinizing agents, include salts such as sodium acetate, sodium carbonate, and/or sodium citrate; organic salts such as benzylamine, trimethylamine, ammonia, urea, and/or ethylenediamine; amino acids such as lysine, cysteine, tyrosine, and/or arginine; as well as combinations of the foregoing.

Acidifiers are capable of releasing protons into the hydrogel or consuming hydroxyl groups from the hydrogel in an amount effective to lower the pH of the hydrogel in which it is placed. Acidifiers include, for example, phosphoric acid, benzoic acid, citric acid, tricarboxylic acids, monobasic sodium phosphate, ammonium chloride, and calcium chloride.

In other embodiments, the buffers above may include alkalizers or acidifiers in a solution formed with any suitable biocompatible solvent.

Since the pKa of a bioactive agent is the pH at which the bioactive agent is about 50% ionized, the surrounding pH affects the degree of ionization of such bioactive agents.

Hydrogels of the present disclosure include those drug molecules or bioactive agents whose pKa fall between the initial pH condition of the hydrogel at in situ formation and the physiologic pH, which is about 7.4. The pH of the hydrogel upon in situ formation may be within a wide range of alkaline or acidic values depending upon the precursors, buffers, and bioactive agents selected. In embodiments, the pH of the hydrogel upon formation is from about 3.0 to about 11.0, in some embodiments from about 5.0 to about 9.5, and in yet other embodiments from about 6.0 to about 9.0. In embodiments, the pH of the hydrogel upon in situ formation is about 8.5.

As noted above, modulation of the release profile of bioactive agents such as drugs can be achieved by changing microenvironmental pH and solubility by adding an appropriate buffer, optionally with an alkalizer or acidizer, without the need for additional excipients. As such, the modulation of the release profile of a bioactive agent can be achieved without any manipulation of the bioactive agent, e.g., microencapsulation, covalent attachment, polymerization, etc.

For example, in embodiments, the bioactive agents may be poorly soluble in water in their free base form. Salts with organic or inorganic low molecular weight acids may be more soluble than the respective free bases. Thus, the pH at which the salt converts to the free base form is dependent on the pKa of the bioactive agent.

An example of such a bioactive agent is bupivacaine. Bupivacaine has a pKa of about 8.1. The salt form of bupivacaine with HCl is illustrated below: 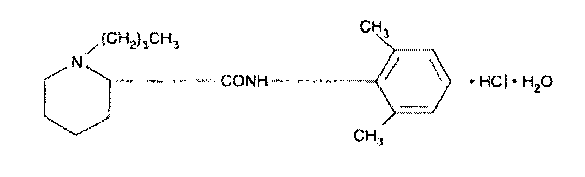 The free base form of bupivacaine, illustrated below, is more hydrophobic than the salt form and thus has a lower aqueous solubility. Thus, the release profile of the bioactive agent can be modulated by modulating the ratio of the salt form to the free base form. The greater the percentage of the bioactive agent in free base form, i.e., the more the equilibrium is shifted toward the free base form, the slower the overall release rate of the bioactive agent, which is solubility driven. 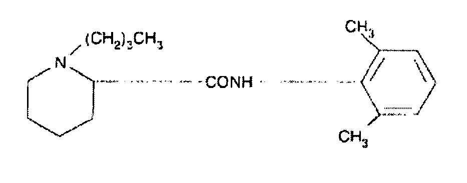 Similarly, in embodiments, the greater the percentage of the bioactive agent in free base form, the lower overall release of the bioactive agent from the hydrogel.

Since the free base form of bupivacaine is less soluble than the ionized hydrochloride salt, slowing the kinetics of the pH transition within the hydrogel, i.e., from 8.5 to 7.4, will favor the free base form and thus slow down release and/or decrease the total amount of release. By keeping the pH environment within the hydrogel higher than 7.4, in embodiments at about 8.1 or above (the pKa of the drug molecule), the bupivacaine would remain in its less soluble, free base form for a longer period of time. Likewise, if the kinetics of the pH transition are sped up, the rate of release is sped up and/or the total amount of release is increased. Accordingly, since the solubility of the free base is less than that of the HCl salt, by keeping the gel at ≥ 8.1 or at least slowing down the pH drop, the bupivacaine will slow down its diffusion out of the hydrogel since the free base converts to the salt as the pH drops.

Accordingly, the release of bupivacaine from a hydrogel having an in situ formation pH above the pKa of bupivacaine can be slowed, and/or the total amount decreased, by including bupivacaine in a buffer with a pH above the pKa of bupivacaine, or by providing a buffer which also includes an alkalizer to provide a pH above the pKa of bupivacaine. The pH of the buffer, optionally enhanced by an alkalizer, slows the pH transition of the hydrogel from alkaline to physiologic and thus slows the conversion of bupivacaine from free base to salt.

For example, if the hydrogel formed in situ has a pH of about 8.5, and a drug such as bupivacaine is added thereto, an alkalizer or acidizer could be added to modify the release profile. As the pKa of the transition of the salt form of bupivacaine into a free-base form is at about 8.1, the kinetics of the transition within the hydrogel, from about 8.5 upon formation of the hydrogel to physiologic pH of about 7.4 after the hydrogel remains in situ, could be slowed by adding an alkalizer such as dibasic sodium phosphate, thereby slowing release and/or decreasing the total amount of bupivacaine released from the hydrogel. Similarly, the addition of an acidizer could increase the kinetics of the transition of the gel to physiologic pH, thereby increasing the rate of release and/or increasing the total amount of bupivacaine released from the hydrogel. Other drugs, having a pKa of from about 7.4 to about 8.5, may similarly be used. Examples of such drugs include, but are not limited to, the following:

| Drug | pKa |
|---|---|
| | |
| Morphine | 7.9 |
| Oxycodone | 8.5 |
| Vancomycin | 7.75 |
| Erythromycin | 8.8 |
| Clonidine | 8.05 |
| Chloroquine | 8.5 |
| Broxuridine | 7.85 |
| Doxorubicin | 8.2 |
| Etidocaine | 7.7 |
| Fluorouracil | 8 |
| Ketamine | 7.7 |
| Lidocaine | 7.9 |
| Methadone | 8.3 |
| Buprenorphine | 8.42 |
| Naloxone | 7.9 |
| Phenytoin | 8.3 |
| Theobromine | 8.8 |
| Chlorcyclizine | 7.8 |
| Chlorprothixine | 8.4 |

Similarly, for a hydrogel with a pH of formation less than physiologic pH of about 7.4, for example 6.5, including a bioactive agent with a pKa of about 6.9, a buffer with a pH less than the pKa of the bioactive agent (6.9) optionally enhanced by an acidizer, could be utilized to decrease the kinetics of the transition of the gel to physiologic pH, thereby keeping the bioactive agent ionized, and increasing the rate of release and/or the total release of the bioactive agent from the gel. For the same gel and bioactive agent, the use of a buffer with optional alkalizer to provide a pH higher than the pKa of the bioactive agent (6.9) could be used to increase the kinetics of the transition of the gel to physiologic pH, thereby making the bioactive agent de-ionized and decreasing the rate of release and/or the total amount of bioactive agent released from the gel. In this example, the assumption is that the de-ionized form of the bioactive agent is less soluble than the ionized form. Other drugs having a lower pKa, in embodiments a pKa of from about 5.5 to about 7.5, which may be utilized include, but are not limited to, the following:

| Drug | pKa |
|---|---|
| | |
| Aminopterin | 5.5 |
| Apomorphine | 7.2 |
| Benzphetamine | 6.6 |
| Benzquinamide | 5.9 |
| 8-Bromotheophylline | 5.5 |
| Capreomycin | 8.2 |
| Carbenoxolone | 6.7 |
| Cephalexin | 5.25 & 7.1 |
| Chlorambucil | 5.8 |
| Chlorothiazide | 6.7 |
| Cimetidine | 6.8 |
| Deserpidine | 6.7 |
| Diphenoxylate | 7.1 |
| Ergotamine | 6.3 & 7.3 |
| Glibenclamide | 6.5 |
| Hexachlorophene | 5.7 |
| Hydroxylamine | 6.0 |
| Indoprofen | 5.8 |
| Kanamycin | 7.2 |
| Levallorphan Tartrate | 6.9 |
| Loxapine | 6.6 |
| Medazepam | 6.2 |
| Methazolamide | 7.3 |
| Methysergide | 6.6 |
| Miconazole | 6.9 |
| Narcotine | 5.9 |
| Nitrofurantoin | 7.2 |
| Norketamine | 6.7 |
| Noscapine | 6.2 |
| Papaverine | 5.9 |
| Pargyline | 6.9 |
| Phenoxypropazine | 6.9 |
| Phtalimide | 7.4 |
| Prazosin | 6.5 |
| Procarbazine | 6.8 |
| Pyrimethamine | 7.2 |
| Reserpine | 6.6 |
| Resorcinol | 6.2 |
| Rolitetracycaline | 7.4 |
| Spectinomycin | 7.0 |
| Sulfadiazine | 6.5 |
| Sulfamerazine | 7.1 |
| Sulfamethazine | 7.4 |
| Sulfathiazole | 7.1 |
| Triamterine | 6.2 |
| Tripolidine | 6.5 |

The following Examples are being submitted to illustrate embodiments of the present disclosure. These Examples are intended to be illustrative only and are not intended to limit the scope of the present disclosure. Also, parts and percentages are by weight unless otherwise indicated. As used herein, "room temperature" refers to a temperature of from about 20 ° C to about 30° C.

### EXAMPLES

### EXAMPLE 1

### Hydrogel Formulation Kit

A kit for preparing a hydrogel contains a dual liquid applicator, plunger cap, spray tips, syringe holder, as well as a syringe containing trilysine dissolved in a phosphate buffer moiety, a syringe containing a borate buffer moiety, a vial containing PEG and FD&C Blue #1 in powder form, and a vial containing Bupivacaine hydrochloride. The aqueous phosphate buffer solution contains monobasic sodium phosphate (NaH₂PO₄) salt along with trilysine having primary amine functional groups dissolved in a ratio yielding a pH of about 4.3. The borate buffer contains dibasic sodium phosphate (NaHPO₄ and has a pH of about 10.0. The PEG is a multiarmed polyethylene glycol electrophilic precursor with succinimidyl ester electrophilic functional groups (specifically, succinimidyl glutarate, SG) on the end of each of four arms (4a) having a total MW of about 20,000 MW polyethylene glycol (sometimes referred to herein as 4a20k SG) in a 1:1 stoichiometric ratio of electrophiles: nucleophiles.

The kit contains instructions for assembling the applicator and forming the first and second precursors. The first precursor is formed when the PEG ester vial contents are reconstituted with the pre-filled syringe of the phosphate buffer solution. The second precursor is formed when the vial containing Bupivacaine hydrochloride is reconstituted with the syringe containing the borate buffer solution. The precursor solutions can be mixed together for rapid polymerization of the materials of the hydrogel almost immediately upon dispensing.

### EXAMPLE 2

### Release of Bupivacaine HCl from a hydrogel tested In Vitro

The release rate of Bupivacaine HCL was tested by the manipulation of the pH of a hydrogel by changing the amount of dibasic sodium phosphate (Na₂HPPO₄) added to a borate buffer moiety.

Upon formation of the hydrogel, the pH was about 8.5. The pH of the release medium was about 7.4 to mimic physiological conditions. The pKa of the transition of the salt form of bupivacaine into a free base form was 8.1. Therefore, after placing the hydrogel within the mimicked physiological environment, the pH of the hydrogel normalized to 7.4 and the bupivacaine reached equilibrium between the HCl salt and the significantly less soluble free base form at a pH of about 8.1. As the pH continued to drop, the bupivacaine released faster due to its increased solubility in low pH aqueous solutions.

Specifically, bupivacaine release was slowed with the addition of a dibasic sodium phosphate (Na₂HPO₄) alkalizer to slow the pH transition from 8.5 to 7.4. It is contemplated that other alkalizers could have been added to the hydrogel as discussed above. Using the formula weight of 142 Daltons, different molar concentrations (0.01 M, 0.05M, 0.1M, and 0.5M) of dibasic Na₂HPO₄ were prepared and evaluated with their incorporation into the gels through in vitro release studies conducted in pH 7.4 isotonic Dulbecco's Phosphate Buffered Saline (DPBS) at 37°C. Samples were prepared using pooled phosphate and borate buffer solutions from twelve hydrogel kits, as described in Example 1 above with the exception that the borate buffer did not contain any dibasic Na₂HPO₄, to eliminate as many variables as possible, such as operator error. Bupivacaine was dissolved in the pooled phosphate buffer at a concentration of 1.5% (w/v) and the various molar concentrations of dibasic sodium phosphate were prepared using the pooled borate buffer as the diluent.

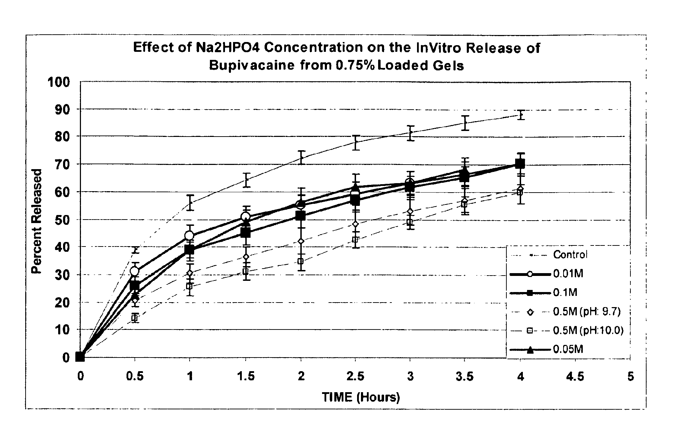

The 50% released time point was selected as the measure by which the release characteristics were evaluated. All samples were prepared with 0.75% drug loading (equal volumes of the 1.5% bupivacaine phosphate buffer and borate buffer) and the in vitro release profiles are presented in FIG. 1. All samples were run in triplicate with the exception of the 0.05M dibasic sodium phosphate study which was run with six replicates. Sampling was conducted every 30 minutes with a 5 ml aliquot removed from the sample and replaced with a 5 ml aliquot of fresh DPBS media. The samples were assayed for drug concentration by HPLC.

Control samples, prepared using pooled buffer without dibasic sodium phosphate, released 50% of their drug load in approximately 48 minutes. The samples prepared with 0.0 1 M, 0.05M, and 0.1M concentrations of dibasic sodium phosphate released 50% of their drug load in approximately 85, 96, and 112 minutes, respectively. Samples prepared with the 0.5M concentration of dibasic sodium phosphate were subdivided in two groups, one whose borate buffer had dropped to a pH of 9.7 when the dibasic sodium phosphate was added and one whose pH was adjusted back to pH 10.0 using 1N sodium hydroxide. The 0.5M samples released 50% in 160 minutes and the 0.5M sample whose pH was adjusted back to 10.0 release 50% in 190 minutes.

The actual percentage released figures were tabulated and are presented in Table 1 below. The concentrations are arranged in ascending order with percentages and the Standard Error of the Mean (SEM) presented.

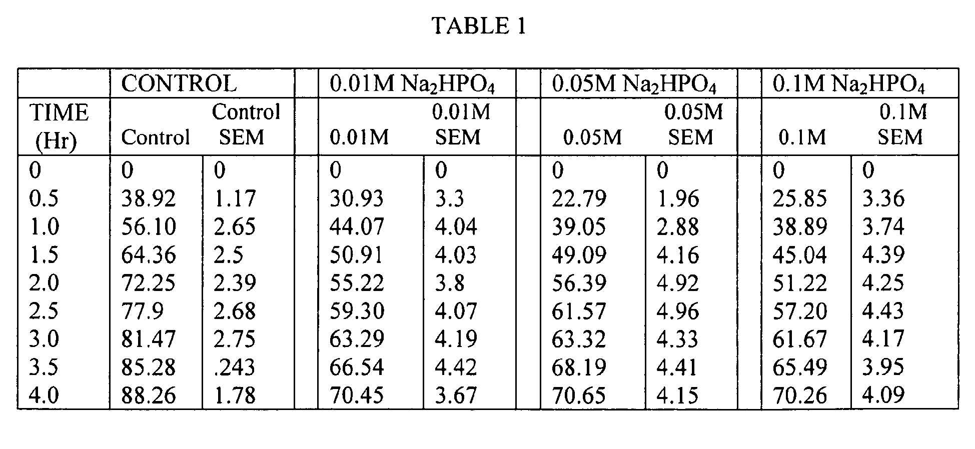

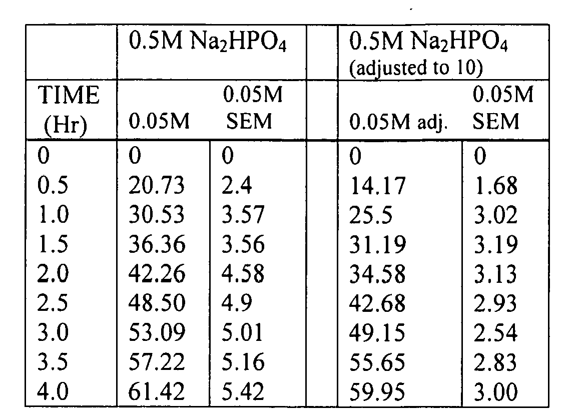

Thus, as shown above, the in vitro release rate of bupivacaine from the hydrogels was controlled by varying the concentrations of dibasic sodium phosphate added to the borate buffer solution. As illustrated in the Figure, there is an asymptotic correlation regarding the release rate and the amount of dibasic sodium phosphate added to the borate solution. There is also a proportional correlation with the rate of release decreasing as the molar concentration increased up to 0.5 M.

All publications, patent applications, and patents mentioned herein are hereby incorporated by reference herein to the extent that they do not contradict the explicit disclosure of this specification. It will be understood that various modifications and changes in form and detail may be made to the embodiments of the present disclosure without departing from the spirit and scope of the disclosure. Various embodiments have been described to provide examples of the hydrogels disclosed herein and are not intended to be limiting; the features and elements of the embodiments may be mixed-and-matched with each other insofar as they result in useable combinations. Therefore, the above description should not be construed as limiting but merely as exemplifications of embodiments of the present disclosure. Those skilled in the art will envision other modifications within the scope and spirit of the present disclosure as defined by the claims appended hereto.

The invention may be described by reference to the following numbered paragraphs:-

A method comprising: contacting a first hydrogel precursor with a first buffer; contacting a second hydrogel precursor with a second buffer; adding a bioactive agent to the first hydrogel precursor, the second hydrogel precursor, or both; and contacting tissue with the first hydrogel precursor and the second hydrogel precursor, wherein the first hydrogel precursor and second hydrogel precursor form a hydrogel upon contact, and wherein the bioactive agent has a pKa between the initial pH of the hydrogel at formation, and the physiologic pH.

The method according to paragraph [0081], wherein forming the hydrogel further comprises a visualization agent.

The method according to paragraph [0081], wherein the first hydrogel precursor possesses first functional groups and the second hydrogel precursor possesses second functional groups.

The method according to paragraph [0083], further comprising: delivering the first and second precursors onto an area of tissue so that a crosslinking reaction is facilitated between the first and second precursors.

The method according to paragraph [0084], wherein the crosslinking reaction leads to gelation of the hydrogel from about 3 seconds to about 1 minute.

The method according to paragraph [0081], wherein the physiologic environment has a pH of about 7.4.

The method according to paragraph [0081], wherein the pH of the formed hydrogel is from about 5.0 to about 9.5.

The method according to paragraph [0081], wherein the pH of the formed hydrogel is about 8.5.

The method according to paragraph [0081], wherein the pKa of the bioactive agent is alkaline.

The method according to paragraph [0081], wherein the first buffer, the second buffer, or both, comprises at least one alkalizer.

The method according to paragraph [0081], wherein the pKa of the bioactive agent is acidic.

The method according to paragraph [0081], wherein the first buffer, the second buffer, or both, comprises at least one acidifier.

A kit for producing a hydrogel comprising: a first precursor; a second precursor; a first buffer for the first precursor; a second buffer for the second precursor; a bioactive agent; and an applicator for substantially simultaneously delivering the bioactive agent, and the first and second precursors.

A hydrogel comprising: a first precursor combined with a first buffer; a second precursor combined with a second buffer; a bioactive agent; and wherein the pKa of the bioactive agent is greater than the pH of the hydrogel, and wherein the pH of the first buffer, the second buffer, or both, is selected to increase or decrease the release of the bioactive agent from the hydrogel.

The hydrogel of paragraph [0094], wherein the increase or decrease in the release of the bioactive agent comprises an increase or decrease in a rate of release of the bioactive agent from the hydrogel.

The hydrogel of paragraph [0094], wherein an amount of bioactive agent released from the hydrogel is increased or decreased at a time from about 30 minutes to about 4 days after administration.

The hydrogel of paragraph [0094], wherein an amount of bioactive agent released from the hydrogel is increased or decreased at a time from about 1 day to about 60 days after administration.

The hydrogel of paragraph [0094], wherein the pH of the first buffer, the second buffer, or both, is greater than the pKa of the bioactive agent, thereby decreasing the release of the bioactive agent from the hydrogel.

The hydrogel of paragraph [0094], wherein the pH of the first buffer, the second buffer, or both, is less than the pKa of the bioactive agent, thereby increasing the release of the bioactive agent from the hydrogel.

A hydrogel comprising: a first precursor combined with a first buffer; a second precursor combined with a second buffer; a bioactive agent; and wherein the pKa of the bioactive agent is less than the pH of the hydrogel, and the pH of the first buffer, the second buffer, or both, is selected to increase or decrease the release of the bioactive agent from the hydrogel.

The hydrogel of paragraph [00100], wherein the increase or decrease in the release of the bioactive agent comprises an increase or decrease in a rate of release of the bioactive agent from the hydrogel.

The hydrogel of paragraph [00100], wherein an amount of bioactive agent released from the hydrogel is increased or decreased at a time from about 30 minutes to about 4 days after administration.

The hydrogel of paragraph [00100], wherein an amount of bioactive agent released from the hydrogel is increased or decreased at a time from about 1 day to about 60 days after administration.

The hydrogel of paragraph [00100], wherein the pH of the first buffer, the second buffer, or both, is less than the pKa of the bioactive agent, thereby decreasing the release of the bioactive agent from the hydrogel.

The hydrogel of paragraph [00100], wherein the pH of the first buffer, the second buffer, or both, is greater than the pKa of the bioactive agent, thereby increasing the release of the bioactive agent from the hydrogel.

## Claims

1. A method comprising:
contacting a first hydrogel precursor with a first buffer;
contacting a second hydrogel precursor with a second buffer;
adding a bioactive agent to the first hydrogel precursor, the second hydrogel precursor, or both; and
contacting tissue with the first hydrogel precursor and the second hydrogel precursor,
wherein the first hydrogel precursor and second hydrogel precursor form a hydrogel upon contact, and wherein the bioactive agent has a pKa between the initial pH of the hydrogel at formation, and the physiologic pH.

2. The method according to claim 1, wherein forming the hydrogel further comprises a visualization agent.

3. The method according to claim 1 or claim 2, wherein the first hydrogel precursor possesses first functional groups and the second hydrogel precursor possesses second functional groups.

4. The method according to claim 3, further comprising:
delivering the first and second precursors onto an area of tissue so that a crosslinking reaction is facilitated between the first and second precursors, preferably wherein the crosslinking reaction leads to gelation of the hydrogel from about 3 seconds to about 1 minutes.

5. The method according to any preceding claim, wherein the physiologic environment has a pH of about 7.4.

6. The method according to any preceding claim, wherein the pH of the formed hydrogel is from about 5.0 to about 9.5.

7. The method according to any preceding claim, wherein the pKa of the bioactive agent is alkaline, preferably wherein the first buffer, the second buffer, or both, comprises at least one alkalizer.

8. The method according to any of claims 1 to 6, wherein the pKa of the bioactive agent is acidic, preferably wherein the first buffer, the second buffer, or both, comprises at least one acidifier.

9. A kit for producing a hydrogel comprising:
a first precursor;
a second precursor;
a first buffer for the first precursor;
a second buffer for the second precursor;
a bioactive agent; and
an applicator for substantially simultaneously delivering the bioactive agent, and the first and second precursors.

10. A hydrogel comprising:
a first precursor combined with a first buffer;
a second precursor combined with a second buffer;
a bioactive agent; and
wherein the pKa of the bioactive agent is greater than the pH of the hydrogel, and wherein the pH of the first buffer, the second buffer, or both, is selected to increase or decrease the release of the bioactive agent from the hydrogel.

11. A hydrogel comprising:
a first precursor combined with a first buffer;
a second precursor combined with a second buffer;
a bioactive agent; and
wherein the pKa of the bioactive agent is less than the pH of the hydrogel, and the pH of the first buffer, the second buffer, or both, is selected to increase or decrease the release of the bioactive agent from the hydrogel.

12. The hydrogel of claim 10 or claim 11, wherein the increase or decrease in the release of the bioactive agent comprises an increase or decrease in a rate of release of the bioactive agent from the hydrogel.

13. The hydrogel of claim 12, wherein an amount of bioactive agent released from the hydrogel is increased or decreased at a time from about 30 minutes to about 4 days after administration; or wherein an amount of bioactive agent released from the hydrogel is increased or decreased at a time from about 1 day to about 60 days after administration.

14. The hydrogel of claim 10, wherein the pH of the first buffer, the second buffer, or both, is greater than the pKa of the bioactive agent, thereby decreasing the release of the bioactive agent from the hydrogel; or wherein the pH of the first buffer, the second buffer, or both, is less than the pKa of the bioactive agent, thereby increasing the release of the bioactive agent from the hydrogel.

15. The hydrogel of claim 11, wherein the pH of the first buffer, the second buffer, or both, is less than the pKa of the bioactive agent, thereby decreasing the release of the bioactive agent from the hydrogel; or wherein the pH of the first buffer, the second buffer, or both, is greater than the pKA of the bioactive agent, thereby increasing the release of the bioactive agent from the hydrogel.
